# FASCICULE DE BREVET EUROPEEN

(11) **EP 3 545 071 B1**
(45) Date de publication et mention de la délivrance du brevet: **09.09.2020**
(21) Numéro de dépôt: 17823035.5
(22) Date de dépôt: 24.11.2017
(51) Int. Cl.: C12M 1/00, C12M 1/12, C12M 3/00

(54) **MODULE POUR PHOTOBIOREACTEUR ET PHOTOBIOREACTEUR ASSOCIE**
MODUL FÜR FOTOBIOREAKTOR UND ZUGEHÖRIGER FOTOBIOREAKTOR
MODULE FOR PHOTOBIOREACTOR AND ASSOCIATED PHOTOBIOREACTOR

(30) Priorité: 25.11.2016 FR 1661496
(43) Date de publication de la demande: 02.10.2019
(73) Titulaire: Centre National de la Recherche Scientifique (CNRS), 75016 Paris (FR); Universite de Nantes, 44000 Nantes (FR); Ecole Nationale Superieure de Chimie de Clermont Ferrand, 63170 Aubière (FR)
(72) Inventeur: PRUVOST, Jérémy, 44250 Saint-Brevin les Pins (FR); LEGRAND, Jack, 44600 Saint Nazaire (FR); CORNET, Jean-François, 63830 Durtol (FR)
(74) Mandataire: Regimbeau
(86) Numéro de dépôt international: PCT/EP2017/080380
(87) Numéro de publication internationale: WO 2018/096107

(56) Documents cités:
- WO-A1-2014/148903
- WO-A1-2015/126002
- US-A1- 2011 076 757
- DATABASE WPI Week 201326 Thomson Scientific, London, GB; AN 2013-B47136 XP002772840, -& WO 2013/100237 A1 (UNIV. CHOSUN IND. ACADEMIC COOP. FOUND.) 4 juillet 2013 (2013-07-04)

## Description

### DOMAINE DE L'INVENTION

La présente invention concerne un système de production de micro-organismes photosynthétiques en suspension dans l'eau.

### ETAT DE LA TECHNIQUE

Un photobioréacteur est un système assurant la production de micro-organismes photosynthétiques en suspension dans l'eau, tels que des plantes pluricellulaires, des petites plantes comme les gamétophytes, les bactéries photosynthétiques, les cyanobactéries, les microalgues eucaryotes, les cellules isolées de macroalgues et les protonemata de mousse.

Cette production se fait par culture, le plus souvent clonale, en milieu aqueux sous éclairage. L'amplification jusqu'à des volumes industriels pouvant atteindre des centaines de mètres cubes s'effectue selon des étapes successives où le volume d'une étape sert à inoculer le volume suivant. Pour récolter la population microbienne et assurer la production de biomasse, le volume de chaque étape peut être renouvelé partiellement tous les jours (culture en continu) ou changé totalement (culture en lots). À ces étapes correspondent des photobioréacteurs de volume croissant.

Les microorganismes photosynthétiques produisent de la biomasse uniquement lorsqu'ils reçoivent une quantité d'énergie lumineuse, appelée irradiance, supérieure à une irradiance minimale Gc. Or, comme une culture d'algues est un milieu absorbant qui atténue la lumière, la lumière diminue avec l'épaisseur de la culture selon une loi exponentielle.

Pour augmenter la productivité en biomasse des systèmes de culture, il convient d'augmenter l'apport en énergie lumineuse et/ou diminuer le volume de culture pour une surface donnée d'éclairement (i.e. augmenter la surface spécifique éclairée). Ainsi, on distingue usuellement la productivité ramenée au volume de culture (i.e. productivité volumique) et la productivité ramenée à la surface éclairée (i.e. productivité surfacique).

Pour pouvoir faire évoluer le volume du photobioréacteur au cours des étapes décrites plus haut, et pour uniformiser l'irradiance reçue par les microorganismes, il a été proposé, dans le document Toshihiko Kondo « Efficient hydrogen production using a multi-layered photobioreactor and a photosynthetic bacterium mutant with reduced pigment », des photobioréacteurs multicouche qui permettent de diluer une lumière incidente forte et de la diffuser uniformément dans le réacteur. Des couches peuvent être progressivement ajoutées au photobioréacteur pour pouvoir faire évoluer le volume du photobioréacteur.

Cependant, les systèmes conçus sur ce principe se heurtent à une limitation majeure, à savoir la tendance à la formation de dépôts, appelés biofilms, sur les parois éclairantes, ce qui induit au fil du temps une diminution de l'intensité lumineuse dans le volume du réacteur et donc l'efficacité de la production. Ce phénomène est d'autant plus important que l'épaisseur de culture (i.e. la distance entre plaques éclairantes) est faible. Le confinement du milieu en effet augmente, ainsi que la concentration en biomasse du fait de l'augmentation de la surface spécifique éclairante qui mène à une productivité volumique plus élevée.

Pour éviter la formation de biofilms, il a été proposé d'insérer des dispositifs de nettoyage mécaniques dans la cavité recevant le milieu de culture, mais des tels dispositifs de nettoyage ne sont pas compatibles avec des cavités de faibles épaisseurs. L'épaisseur de culture doit donc en pratique présenter une épaisseur minimale de quelques centimètres, typiquement supérieure à 1 cm, ce qui ne permet pas l'obtention d'une productivité volumique élevée.

Au final, ces systèmes conçus sur le principe de plaques immergés éclairantes présentent des productivités en volume faibles, et une tendance forte à l'encrassement.

En termes de technologies permettant une productivité volumique élevée, on peut citer à titre d'exemple le document FR2950899 qui décrit un photobioréacteur comprenant un panneau éclairant incliné d'une pente moyenne suivant une direction d'inclinaison sur laquelle ruisselle une solution. Cela permet d'obtenir une épaisseur minimale de quelques millimètres et donc une productivité volumique élevée. Ainsi, le panneau éclairant n'est pas en contact avec la culture ce qui permet d'éviter les biofilms. Une telle solution n'est, en revanche, pas compatible avec un photobioréacteur modulaire à éclairage interne. Le document WO2014/148903 décrit un photobioréacteur avec une pluralité de panneaux verticales avec un dispositif d'injection de gaz.

### EXPOSE DE L'INVENTION

Un but de l'invention est de proposer une solution pour à la fois permettre une évolution du volume du photobioréacteur tout en maintenant une production en volume intensifiée et efficace de biomasse.

Ce but est atteint dans le cadre de la présente invention grâce à un module pour photobioréacteur adapté pour être assemblé à un module adjacent identique, comprenant :
- un panneau présentant une première face et une deuxième face,, opposée à la première face, la première face délimitant, avec la deuxième face du panneau adjacent, une cavité propre à contenir un milieu de culture lorsque les modules sont assemblés, la cavité présentant une épaisseur comprise entre 1 millimètre et 2 centimètres, et
- un dispositif d'injection de gaz, propre à injecter des bulles de gaz dans le milieu de culture contenu dans la cavité , de sorte que les bulles remontent le long des faces des panneaux.

L'épaisseur de culture, définie par l'espaces entre plaques est très faible, ce qui permet d'obtenir des productivités en volume élevées. La formation de biofilms est évitée par un bullage efficace entre les deux plaques. En effet, du fait de la faible épaisseur de la cavité, les bulles de gaz remontant le long des faces des panneaux réalisant au passage un nettoyage mécanique desdits panneaux. L'injection de gaz, nécessaire à la culture des microorganismes (apport d'éléments nutritifs), permet en outre de favoriser la mise en mouvement du milieu de culture, ce qui permet d'uniformiser l'irradiation des microorganismes présents dans la cavité.

Le module est également conçu pour garantir des productivités en volume et en surface constantes. Le caractère modulaire du photobioréacteur permet, en effet d'augmenter la capacité de production de façon simple et linéaire, par ajout de modules maintenant une exposition optimale des microorganismes à la lumière. Les modules peuvent être ajoutés au photobioréacteur pour pouvoir faire évoluer le volume du photobioréacteur de quelques litres jusqu'à plusieurs m3. Du fait de la faible épaisseur de la cavité et de l'éclairage sur les deux côtés (l'épaisseur de culture est donc égale à la moitié de l'épaisseur de la cavité), un tel module permet de fabriquer un photobioréacteur présentant une haute productivité volumique, et ce quel que soit le nombre de modules assemblés.

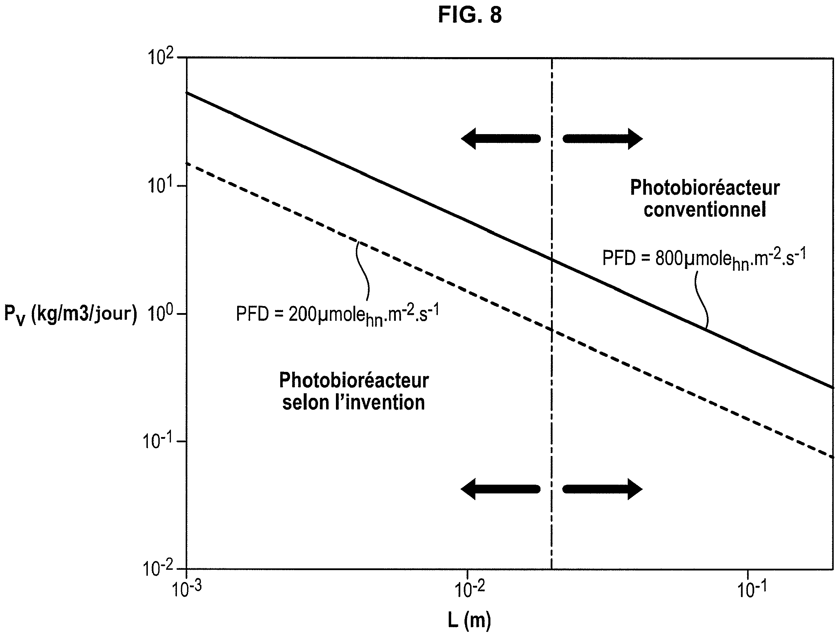

La figure 8 illustre la productivité volumique par jour Pv (tracé en log) en fonction de l'épaisseur de culture L, d'une part pour un flux lumineux (PFD) de 800 µmole.m-1.s-1 (trait plein), et d'autre part pour un flux lumineux (PFD) de 200 µmole.m-1.s-1 (trait en pointillé). Ce graphique montre que l'invention permet de produire plus de 10 fois plus que l'art antérieur.

L'invention est avantageusement complétée par les caractéristiques suivantes, prises individuellement ou en l'une quelconque de leurs combinaisons techniquement possibles.
- le module comprend un dispositif d'éclairage, propre à éclairer le milieu de culture contenu dans la cavité, le dispositif d'éclairage comprenant une nappe de fibres optiques tressées ou une plaque en verre diffusant.
- le panneau comprend deux plaques transparentes entre lesquelles s'étend le dispositif d'éclairage ;
- chaque plaque transparente est en polyméthacrylate de méthyle, verre ou tout autre matériau transparent ;
- le dispositif d'injection de gaz comprend des canaux d'injection de gaz creusés ou rapportés dans l'une des plaques transparentes ;
- le panneau comprend une ouverture autorisant une communication de fluide entre cavités lorsque plusieurs modules sont assemblés ;
- la première et/ou la deuxième face du panneau présente(nt) un renfoncement pour former la cavité ;
- le module comprend un joint d'étanchéité agencé pour créer un contact étanche entre les modules ;
- le module comprend un cadre s'étendant le long de bords du panneau pour rigidifier le panneau.

L'invention concerne également un photobioréacteur, comprenant un assemblage de plusieurs modules tels que décrits plus haut, chaque module définissant, avec un module adjacent, une cavité propre à contenir le milieu de culture.

Le photobioréacteur comprend un assemblage de plusieurs modules et une unité de commande configurée pour commander l'injection des bulles de gaz dans le milieu de culture contenu dans la cavité, alternativement sur une première section de la largeur de la cavité, puis sur une deuxième section de la largeur de la cavité.

Le photobioréacteur comprend un bâti comprenant des rails de guidage sur lesquels les modules sont en appui de manière à maintenir un positionnement relatif des modules entre eux.

Le photobioréacteur comprend un dispositif de pressage de l'assemblage pour maintenir les modules en contact étanche les uns avec les autres.

Le dispositif de pressage comprend une première plaque d'extrémité et une deuxième plaque d'extrémité, entre lesquelles sont disposés les modules, et un actionneur propre à exercer sur la deuxième plaque une pression tendant rapprocher la deuxième plaque de la première plaque.

Le photobioréacteur comprend un dispositif de régulation de température du milieu de culture.

Les modules incluent un module instrumenté, le module instrumenté comprenant un ou plusieurs capteurs en contact avec le milieu de culture.

### DESCRIPTION DES FIGURES

D'autres objectifs, caractéristiques et avantages sortiront de la description détaillée qui suit en référence aux dessins donnés à titre illustratif et non limitatif parmi lesquels :
- la figure 1 représente un photobioréacteur conforme à un mode de réalisation de l'invention ;
- la figure 2 représente un module conforme à un mode de réalisation de l'invention ;
- la figure 3 est une vue en coupe d'un module conforme à un mode de réalisation de l'invention ;
- la figure 4 est une vue en coupe d'un assemblage de modules ;
- la figure 5 est une vue en coupe d'un module conforme à un mode de réalisation de l'invention ;
- la figure 6 est une vue éclatée d'un photobioréacteur conforme à un mode de réalisation de l'invention ;
- la figure 7 est une vue en coupe d'un assemblage de modules comportant un module d'instrumentation ;
- la figure 8 illustre la productivité volumique par jour (tracé en log) en fonction de l'épaisseur de culture.

### DESCRIPTION DETAILLEE DE L'INVENTION

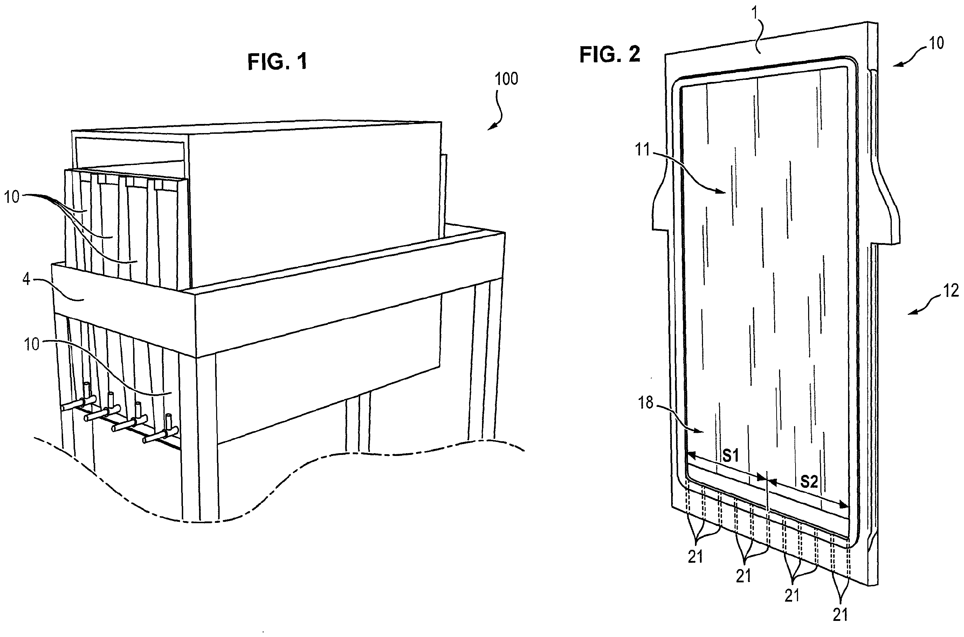

En référence à la figure 1, un photobioréacteur 100, comprend un bâti 4 et une série de modules 1.

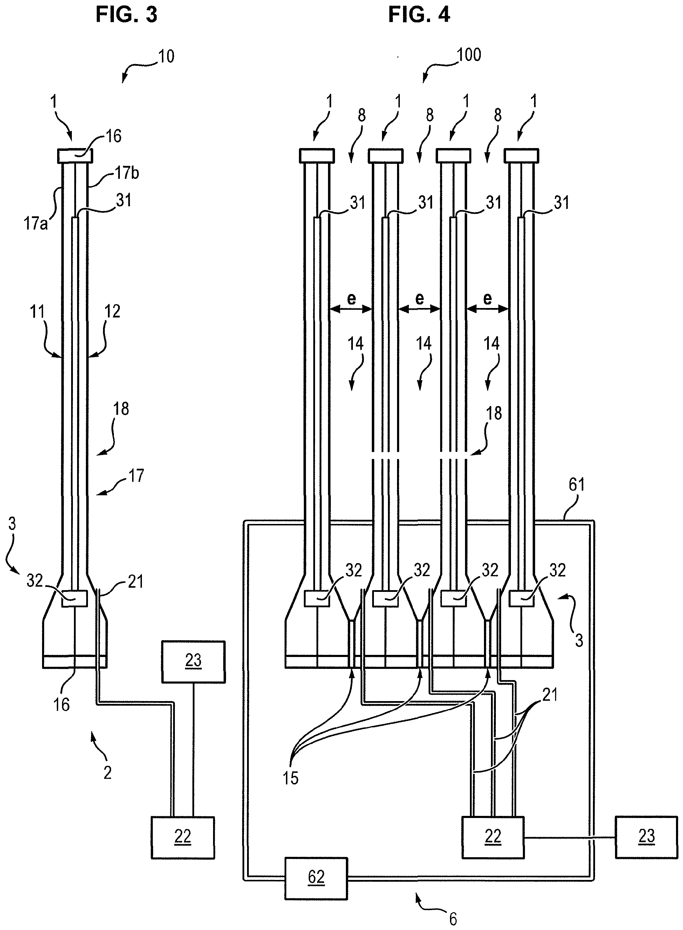

En référence à la figure 4, chaque module 10 pour photobioréacteur est adapté pour être assemblé à un module adjacent identique de manière à définir, avec celui-ci, une cavité 14 présentant une ouverture 8 en partie haute et propre à contenir un milieu de culture. Chaque module 10, comprend un panneau 1, un dispositif d'injection de gaz 21.

La distance entre deux panneaux 1 définie l'épaisseur de la cavité 14, tandis que la largeur du panneau 1 définie la largeur de la cavité 14, et la hauteur du panneau 1 la hauteur de la cavité 14.

En référence aux figures 2, 3 et 4, chaque panneau 1 présentent une première face 11 et une deuxième face 12, opposée à la première face 11. La première face 11 délimite, avec la deuxième face 12 du panneau 1 adjacent, une cavité 14 propre à contenir un milieu de culture lorsque les modules 10 sont assemblés. La cavité 14 présente une épaisseur e comprise entre 1 millimètre et 2 centimètres, de préférence comprise entre 1 millimètre et 9 millimètres, par exemple 6 millimètres.

En référence à la figure 3, chaque panneau 1 comprend deux plaques 17a, 17b transparentes. Les plaques 17a, 17b transparentes sont faites avec des matériaux tels que du verre ou des plastiques rigides (par exemple du méthacrylate de méthyle (PMMA), ou du polycarbonate) ou avec des films souples en plastique (par exemple du polyéthylène, polyuréthane, ou du chlorure de vinyle - PVC). Chaque module 10 comprend un cadre 16 s'étendant le long de bords du panneau pour rigidifier le panneau. La première face 11 et la deuxième face 12 du panneau 1 présentent un renfoncement 17 pour former la cavité 14. Plus précisément, chacune des plaques 17a et 17b est formée en une seule pièce unique de matériau, le renfoncement 17 étant formé dans la plaque 17a et/ou dans la plaque 17b.

En référence à la figure 4, chaque module 10 comprend un joint 15 d'étanchéité agencé pour créer un contact étanche entre les modules 10 lorsqu'ils sont assemblés.

En référence à la figure 3, le dispositif d'injection de gaz 2 est propre à injecter des bulles de gaz dans le milieu de culture contenu dans la cavité 14, de sorte que les bulles remontent le long des faces 11 et 12 des panneaux 1. Le gaz injecté contient du dioxyde de carbone et est typiquement de l'air ou un gaz issu d'une réaction et collecté en sortie d'usine.

Le dispositif d'injection de gaz 2 comprend des canaux d'injection de gaz 21 répartis sur la largeur de la cavité 14 en partie basse des panneaux 1. Les canaux d'injection de gaz 21 sont creusés dans l'une des plaques transparentes 17a, 17b de chaque module 10 ou ménagés entre les deux plaques transparentes 17a, 17b. Le dispositif d'injection de gaz 2 comprend typiquement un réservoir 22 contenant le gaz à injecter sous pression, le réservoir comportant une sortie en communication avec les canaux d'injection de gaz 21.

Le dispositif d'injection de gaz 2 injecte le gaz chargé en dioxyde de carbone nécessaire aux microorganismes via les canaux d'injection de gaz 21 dans la cavité 14. Le gaz injecté forme des bulles qui remontent le long des faces 11, 12 des panneaux 1. En remontant les bulles se chargent en oxygène photosynthétique généré par les microorganismes. Le gaz chargé en oxygène photosynthétique généré par les microorganismes est évacué par une ouverture 8 ménagée en partie haute des panneaux.

Le dispositif d'injection de gaz 2 permet de renouveler le dioxyde de carbone consommé par les microorganismes et d'éliminer l'oxygène photosynthétique généré par les microorganismes. En effet, une concentration trop importante en oxygène et trop faible en dioxyde de carbone inhibe la réaction photosynthétique.

En remontant le long des faces des panneaux 11 et 12, les bulles effectuent un nettoyage mécanique de la paroi intérieure de la cavité 14 qui empêche le développement d'un biofilm qui diminuerait l'intensité lumineuse dans le volume du réacteur.

L'injection de gaz permet en outre de favoriser la mise en mouvement du milieu de culture, afin d'améliorer l'uniformité de l'irradiation des microorganismes.

Le dispositif d'injection de gaz 2 est avantageusement adapté pour injecter du gaz de manière pulsée et séquentielle de manière à augmenter l'effet de cisaillement sur le biofilm en formation.

Une unité de commande 23 configurée pour commander l'injection des bulles de gaz dans le milieu de culture contenu dans la cavité 14, commande l'injection des bulles alternativement les canaux d'injection de gaz 21 répartis sur une première section S1 de la largeur de la cavité 14, puis les canaux d'injection de gaz 21 répartis sur une deuxième section S2 de la largeur de la cavité 14.

L'unité de commande 23 peut également être configurée pour commander alternativement les canaux d'injection de gaz 21 répartis sur la première section S1 d'un premier groupe de cavités 14, puis les canaux d'injection de gaz 21 répartis sur la deuxième section S2 d'un second groupe de cavités 14.

L'unité de commande 23 peut également être configurée pour commander alternativement les canaux d'injection de gaz 21 répartis sur la première section S1 d'un premier groupe de cavités 14 et les canaux d'injection de gaz 21 répartis sur la deuxième section S2 d'un second groupe de cavités 14, puis les canaux d'injection de gaz 21 répartis sur la seconde section S2 du premier groupe de cavités 14 et les canaux d'injection de gaz 21 répartis sur la première section S1 du second groupe de cavités 14.

La première section S1 de la largeur de la cavité 14, est typiquement complémentaire de la deuxième section S2 de la largeur de la cavité 14.

A cet effet, l'unité de commande 23 commande l'injection des bulles selon la séquence suivante :
injection des bulles par les canaux d'injection de gaz 21 répartis sur une première section S1 de la largeur de la cavité 14,
injection des bulles par les canaux d'injection de gaz 21 répartis sur une deuxième section S2 de la largeur de la cavité 14.

La séquence d'injection peut en outre comporter une étape d'injection des bulles simultanément par les canaux d'injection de gaz 21 répartis sur une première section S1 de la largeur de la cavité 14, et par ceux repartis sur la deuxième section S2 de la largeur de la cavité 14.

Les inventeurs ont montré que le fait d'injecter des bulles de gaz alternativement sur une section S1 de la largeur de la cavité 14 puis sur l'autre section S2 de la largeur de la cavité 14 permettait d'améliorer considérablement l'efficacité du nettoyage.

En effet, l'injection des bulles entraine le liquide en mouvement ascendant. Ce mouvement ascendant est compensé par un déplacement descendant de liquide. Or ce déplacement descendant de liquide perturbe l'effet de cisaillement des bulles.

Quand des bulles de gaz sont injectées sur une section de la largeur de la cavité 14 seulement, les bulles entrainent le liquide en mouvement ascendant sur ladite section de la largeur de la cavité 14 et le liquide redescend au niveau de la section complémentaire de la largeur de la cavité 14 en formant un mouvement cyclique. Ainsi le déplacement descendant de liquide ne perturbe pas l'effet de cisaillement des bulles. Le nettoyage de la section complémentaire de la largeur de la cavité 14 est ensuite réalisé en injectant des bulles de gaz au niveau de la section complémentaire de la largeur de la cavité 14. Ainsi, la cavité 14 est finalement nettoyée sur toute sa largeur.

Par ailleurs, le dispositif d'injection de gaz 2 est avantageusement adapté pour former des bulles ayant une taille du même ordre de grandeur que l'épaisseur e pour augmenter l'effet de cisaillement sur le biofilm en formation.

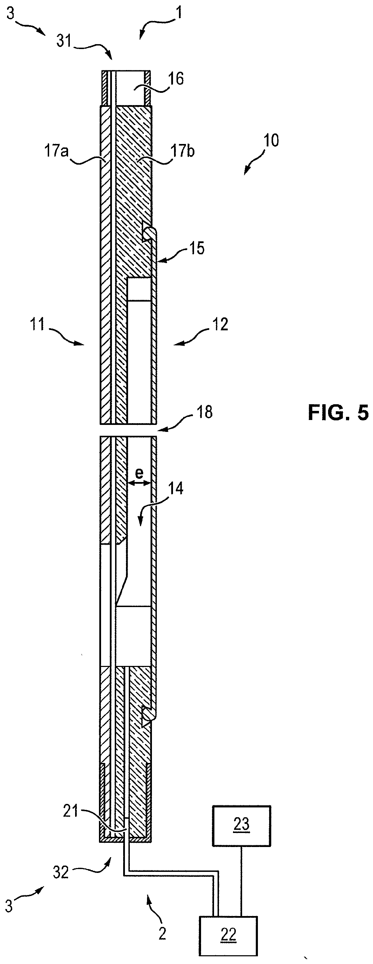

En référence à la figure 5, chaque module 10 comprend un dispositif d'éclairage 3, propre à éclairer le milieu de culture contenu dans la cavité 14, le dispositif d'éclairage 3 comprenant une source lumineuse 32 et un dispositif optique de diffusion 31.

La source lumineuse 32 est typiquement une diode électroluminescente. Elle émet typiquement en lumière blanche dans un domaine spectrale adapté pour améliorer la conversion photosynthétique.

La source lumineuse 32 peut être commune à tous les modules 10. Notamment, la source lumineuse 32 peut être la lumière solaire. Dans ce cas, la source lumineuse 32 comporte un système de captation du flux solaire et de transmission du flux jusqu'aux dispositifs optiques de diffusion 31.

Le dispositif optique de diffusion 31 s'étend entre les deux plaques 17a, 17b transparentes. L'éclairage interne permet une absorption totale du flux émis, une maîtrise des conditions d'atténuation de lumière et donc de la conversion biologique, et une faible consommation énergétique.

Le dispositif optique de diffusion 31 est typiquement un dispositif à diffusion latérale comme une nappe de fibres optiques tissées à diffusion latérale ou une plaque de verre diffusante, ou une feuille polymère fine dans laquelle sont intégrées des rubans de diodes électroluminescentes LED.

En référence à la figure 5, le panneau 1 comprend une ouverture 18 autorisant une communication de fluide entre cavités 14 lorsque plusieurs modules 10 sont assemblés. Ainsi, les modules 10 peuvent être assemblés entre eux de manière à ce que les cavités 14 soient en communication fluidique.

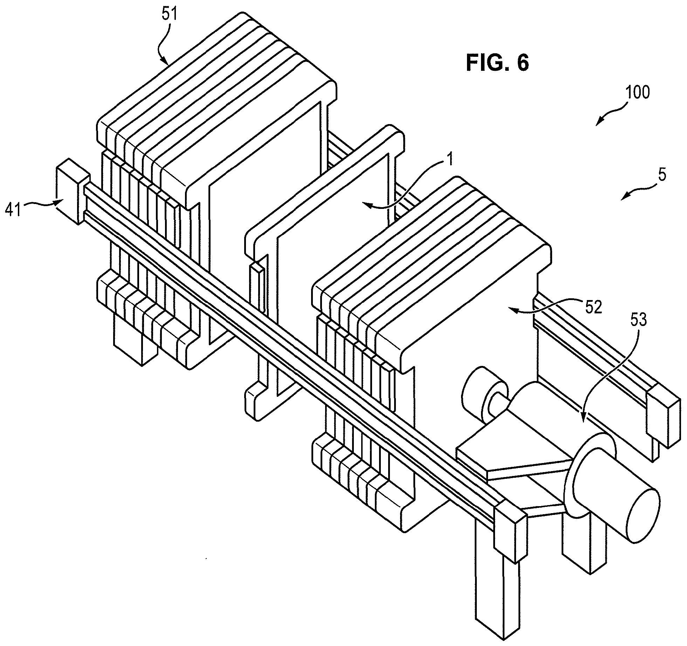

En référence à la figure 6, le bâti 4 comprend typiquement des rails de guidage 41 sur lesquels les modules 10 sont en appui de manière à maintenir un positionnement relatif des modules 10 entre eux. Le photobioréacteur 100 comprend en outre un dispositif de pressage 5 de l'assemblage pour maintenir les modules 10 en contact étanche les uns avec les autres. Le dispositif de pressage 5 comprend une première plaque d'extrémité 51 et une deuxième plaque d'extrémité 52, entre lesquelles sont disposés les modules 10, et un actionneur 53 propre à exercer sur la deuxième plaque 52 une pression tendant rapprocher la deuxième plaque 52 de la première plaque 51. La pression exercée permet d'assurer l'étanchéité entre les différents modules 10. L'actionneur 53 est typiquement un vérin hydraulique actionné par une pompe (centrifuge ou à membrane par exemple).

En référence à la figure 4, le photobioréacteur 100 comprend en outre un dispositif 6 de régulation de température du milieu de culture, pour maintenir les températures de culture à une température optimale dans le photobioréacteur. En effet, une culture dense absorbe le rayonnement infrarouge, ce qui peut conduire à des températures létales aux microorganismes si l'excès de chaleur n'est pas évacué.

A cet effet, le dispositif 6 de régulation de température comprend par exemple un circuit de circulation comprenant un tube de circulation 61 d'un liquide de refroidissement et un module de refroidissement 62, le tube de circulation 61 s'étendant à travers le module de refroidissement 62. Le tube de circulation 61 s'étend par exemple à travers les modules 10 ou à travers les plaques 17a, 7b. Le liquide de refroidissement peut par exemple être de l'eau.

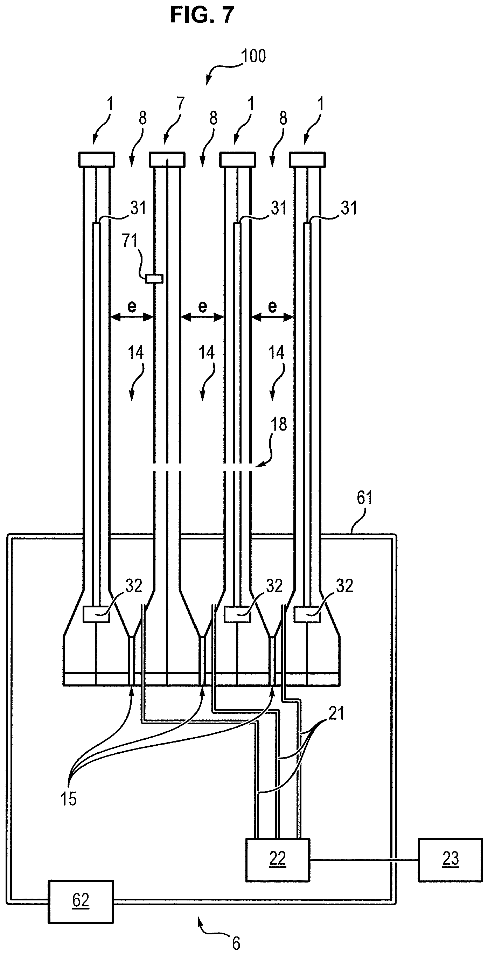

En référence à la figure 7, le photobioréacteur 100 comporte en outre au moins un module instrumenté 7. Le module instrumenté 7 est semblable aux autres modules 10 et comprend un ou plusieurs capteurs 71, comme par exemple un capteur de température, ou un capteur de concentration d'oxygène, en contact avec le milieu de culture. Les informations issues de ces capteurs permettent de contrôler les conditions dans la cavité et notamment la température, l'éclairement, et la composition de la solution afin d'optimiser la productivité de la culture.

## Revendications

1. Module (10) pour photobioréacteur adapté pour être assemblé à un module (10) adjacent identique, comprenant :
- un panneau (1) présentant une première face (11) et une deuxième face (12), opposée à la première face (11), la première face (11) délimitant, avec la deuxième face (12) du panneau (1) adjacent, une cavité (14) propre à contenir un milieu de culture lorsque les modules (10) sont assemblés, la cavité (14) présentant une épaisseur (e) comprise entre 1 millimètre et 2 centimètres, le panneau (1) comprenant deux plaques (17a, 17b) transparentes, et
- un dispositif d'injection de gaz (2), propre à injecter des bulles de gaz dans le milieu de culture contenu dans la cavité (14), de sorte que les bulles remontent le long des faces (11, 12) des panneaux (1),
dans lequel le dispositif d'injection de gaz (2) comprend des canaux d'injection de gaz (21) creusés ou rapportés dans l'une des plaques transparentes (17a, 17b).

2. Module (10) selon la revendication 1, comprenant un dispositif d'éclairage (3), propre à éclairer le milieu de culture contenu dans la cavité (14), le dispositif d'éclairage (3) comprenant une nappe (31) de fibres optiques tissées ou une plaque diffusante ou une plaque dans laquelle sont intégrés des sources lumineuses de type diode électroluminescente.

3. Module (10) selon la revendication 2, dans lequel le dispositif d'éclairage (3) s'étend entre les deux plaques transparentes (17a, 17b).

4. Module (10) selon l'une des revendications 1 à 3, dans lequel chaque plaque (17a, 17b) transparente est en polyméthacrylate de méthyle, verre ou tout autre matériau transparent.

5. Module (10) selon l'une des revendications qui précèdent, dans lequel le panneau (1) comprend une ouverture (18) autorisant une communication de fluide entre cavités lorsque plusieurs modules (10) sont assemblés.

6. Module (10) selon l'une des revendications 1 à 5, dans lequel la première et/ou la deuxième face du panneau (1) présente(nt) un renfoncement (17) pour former la cavité.

7. Module (10) selon l'une des revendications 1 à 6, comprenant un joint (15) d'étanchéité agencé pour créer un contact étanche entre les modules (10).

8. Module (10) selon l'une des revendications 1 à 7, comprenant un cadre (16) s'étendant le long de bords du panneau pour rigidifier le panneau.

9. Photobioréacteur (100), comprenant un assemblage de plusieurs modules (10) selon l'une des revendications 1 à 8, chaque module (10) définissant, avec un module (10) adjacent, une cavité (14) propre à contenir le milieu de culture.

10. Photobioréacteur (100), comprenant un assemblage de plusieurs modules (10) selon la revendication précédente, comportant en outre une unité de commande (23) configurée pour commander l'injection des bulles de gaz dans le milieu de culture contenu dans les cavités (14), alternativement sur une première section (S1) de la largeur de la cavité (14), puis sur une deuxième section (S2) de la largeur de la cavité (14).

11. Photobioréacteur (100) selon l'une des revendications 9 ou 10, comprenant un bâti (4) comprenant des rails de guidage (41) sur lesquels les modules (10) sont en appui de manière à maintenir un positionnement relatif des modules (10) entre eux.

12. Photobioréacteur (10) selon l'une des revendications 9 à 11, comprenant un dispositif de pressage (5) de l'assemblage pour maintenir les modules (10) en contact étanche les uns avec les autres.

13. Photobioréacteur (10) selon la revendication 12, dans lequel le dispositif de pressage (5) comprend une première plaque d'extrémité (51) et une deuxième plaque d'extrémité (52), entre lesquelles sont disposés les modules (10), et un actionneur (53) propre à exercer sur la deuxième plaque (52) une pression tendant rapprocher la deuxième plaque (52) de la première plaque (51).

14. Photobioréacteur (10), selon l'une des revendications 9 à 13, comprenant un dispositif (6) de régulation de température du milieu de culture.

15. Photobioréacteur (10), selon l'une des revendications 9 à 14, dans lequel les modules (10) incluent un module instrumenté (7), le module instrumenté (7) comprenant un ou plusieurs capteurs (71) en contact avec le milieu de culture.

## Patentansprüche

1. Modul (10) für Photobioreaktor, das zur Verbindung mit einem identischen benachbarten Modul (10) geeignet ist, umfassend:
- eine Platte (1), die eine erste Fläche (11) und eine zweite Fläche (12) aufweist, die der ersten Fläche (11) gegenüberliegt, wobei die erste Fläche (11) mit der zweiten Fläche (12) der benachbarten Platte (1) einen Hohlraum (14) begrenzt, der imstande ist, ein Kulturmedium zu enthalten, wenn die Module (10) verbunden sind, wobei der Hohlraum (14) eine Dicke (e) aufweist, die zwischen 1 Millimeter und 2 Zentimeter liegt, wobei die Platte (1) zwei transparente Tafeln (17a, 17b) umfasst, und
- eine Gaseinleitungsvorrichtung (2), die imstande ist, Gasblasen in das in dem Hohlraum (14) enthaltene Kulturmedium derart einzuleiten, dass die Blasen entlang der Flächen (11, 12) der Platten (1) aufsteigen,
wobei die Gaseinleitungsvorrichtung (2) Gaseinleitungskanäle (21) umfasst, die in einer der transparenten Tafeln (17a, 17b) eingearbeitet oder angebracht sind.

2. Modul (10) nach Anspruch 1, umfassend eine Beleuchtungsvorrichtung (3), die imstande ist, das in dem Hohlraum (14) enthaltene Kulturmedium zu beleuchten, wobei die Beleuchtungsvorrichtung (3) eine aus optischen Fasern gewebte (31) Matte oder eine Diffusionstafel oder eine Tafel umfasst, in die Lichtquellen vom Typ LED integriert sind.

3. Modul (10) nach Anspruch 2, wobei sich die Beleuchtungsvorrichtung (3) zwischen den zwei transparenten Tafeln (17a, 17b) erstreckt.

4. Modul (10) nach einem der Ansprüche 1 bis 3, wobei jede transparente Tafel (17a, 17b) aus Polymethylmethacrylat, Glas oder jedem anderen transparenten Material ist.

5. Modul (10) nach einem der vorangehenden Ansprüche, wobei die Platte (1) eine Öffnung (18) umfasst, die eine Fluidkommunikation zwischen Hohlräumen gestattet, wenn mehrere Module (10) verbunden sind.

6. Modul (10) nach einem der Ansprüche 1 bis 5, wobei die erste und/oder die zweite Fläche der Platte (1) eine Verstärkung (17) aufweist/aufweisen, um den Hohlraum zu bilden.

7. Modul (10) nach einem der Ansprüche 1 bis 6, umfassend eine Dichtung (15), die ausgebildet ist, um einen dichten Kontakt zwischen den Modulen (10) herzustellen.

8. Modul (10) nach einem der Ansprüche 1 bis 7, umfassend einen Rahmen (16), der sich entlang von Rändern der Platte erstreckt, um die Platte zu versteifen.

9. Photobioreaktor (100), umfassend eine Anordnung aus mehreren Modulen (10) nach einem der Ansprüche 1 bis 8, wobei jedes Modul (10) mit einem benachbarten Modul (10) einen Hohlraum (14) definiert, der imstande ist, das Kulturmedium zu enthalten.

10. Photobioreaktor (100), umfassend eine Anordnung aus mehreren Modulen (10) nach vorangehendem Anspruch, aufweisend ferner eine Steuereinheit (23), die ausgelegt ist, um die Einleitung der Gasblasen in das in den Hohlräumen (14) enthaltene Kulturmedium alternativ über einen ersten Abschnitt (S1) der Breite des Hohlraums (14), dann über einen zweiten Abschnitt (S2) der Breite des Hohlraums (14) zu steuern.

11. Photobioreaktor (100) nach einem der Ansprüche 9 oder 10, umfassend ein Gestell (4), das Führungsschienen (41) umfasst, auf denen sich die Module (10) derart abstützen, dass eine relative Positionierung der Module (10) untereinander aufrechterhalten wird.

12. Photobioreaktor (10) nach einem der Ansprüche 9 bis 11, umfassend eine Pressvorrichtung (5) der Anordnung, um die Module (10) in dichtem Kontakt miteinander zu halten.

13. Photobioreaktor (10) nach Anspruch 12, wobei die Pressvorrichtung (5) eine erste Endtafel (51) und eine zweite Endtafel (52) umfasst, zwischen denen die Module (10) angeordnet sind, und einen Aktuator (53), der imstande ist, auf die zweite Tafel (52) einen Druck auszuüben, der bewirkt, dass sich die zweite Tafel (52) der ersten Tafel (51) annähert.

14. Photobioreaktor (10) nach einem der Ansprüche 9 bis 13, umfassend eine Vorrichtung zur Regulierung der Temperatur (6) des Kulturmediums.

15. Photobioreaktor (10) nach einem der Ansprüche 9 bis 14, wobei die Module (10) ein instrumentiertes Modul (7) enthalten, wobei das instrumentierte Modul (7) einen oder mehrere Sensoren (71) im Kontakt mit dem Kulturmedium umfasst.

## Claims

1. Photobioreactor module (10) adapted to be assembled with an identical adjacent module (10), comprising:
- a panel (1) having a first side (11) and a second side (12), opposite the first side (11), the first side (11) delimiting, with the second side (12) of the adjacent panel (1), a cavity (14) suitable for containing a culture medium when the modules (10) are assembled, the cavity (14) having a thickness (e) comprised between 1 millimeter and 2 centimeters, the panel (1) comprising two transparent plates (17a, 17b), and
- a gas injection device (2), suitable for injecting gas bubbles into the culture medium contained in the cavity (14), so that the bubbles rise up the sides (11, 12) of the panels (1),
wherein the gas injection device (2) comprises gas injection channels (21) cut or inserted into one of the transparent plates (17a, 17b).

2. Module (10) according to claim 1, comprising an illumination device (3), suitable for illuminating the culture medium contained in the cavity (14), the illumination device (3) comprising a woven optical fiber web (31) or a diffuser plate or a plate into which light-emitting diode type light sources are integrated.

3. Module (10) according to claim 2, wherein the illumination device (3) extends between the two transparent plates (17a, 17b).

4. Module (10) according to one of claims 1 to 3, wherein each transparent plate (17a, 17b) is made of poly(methyl methacrylate), glass or any other transparent material.

5. Module (10) according to one of the preceding claims, wherein the panel (1) comprises an opening (18) allowing fluid communication between cavities when several modules (10) are assembled.

6. Module (10) according to one of claims 1 to 5, wherein the first and/or the second side of the panel (1) have/has a recess (17) to form the cavity.

7. Module (10) according to one of claims 1 to 6, comprising a gasket (15) arranged to create a tight contact between the modules (10).

8. Module (10) according to one of claims 1 to 7, comprising a frame (16) extending along the edges of the panel to stiffen the panel.

9. Photobioreactor (100), comprising an assembly of several modules (10) according to one of claims 1 to 8, each module (10) defining, with an adjacent module (10), a cavity (14) suitable for containing the culture medium.

10. Photobioreactor (100), comprising an assembly of several modules (10) according to the preceding claim, further comprising a control unit (23) configured to control the injection of gas bubbles into the culture medium contained in the cavities (14), alternately over a first section (S1) of the width of the cavity (14), and then over a second section (S2) of the width of the cavity (14).

11. Photobioreactor (100) according to one of claims 9 and 10, comprising a frame (4) comprising guide rails (41) on which the modules (10) are supported so as to keep the modules (10) in position relative to each other.

12. Photobioreactor (10) according to one of claims 9 to 11, comprising a device (5) for pressing the assembly to keep the modules (10) in tight contact with each other.

13. Photobioreactor (10) according to claim 12, wherein the pressing device (5) comprises a first end plate (51) and a second end plate (52), between which the modules (10) are arranged, and an actuator (53) suitable for exerting on the second plate (52) a pressure which tends to bring the second plate (52) closer to the first plate (51).

14. Photobioreactor (10), according to one of claims 9 to 13, comprising a device (6) for controlling the temperature of the culture medium.

15. Photobioreactor (10), according to one of claims 9 to 14, wherein the modules (10) include an instrumented module (7), the instrumented module (7) comprising one or more sensors (71) in contact with the culture medium.
